(19) Europäisches Patentamt
European Patent Office
Office européen des brevets

(11) Publication number: **0 488 047 A1**

(12) **EUROPEAN PATENT APPLICATION**

(21) Application number: **91119844.8**

(51) Int. Cl.[5]: **C07D 311/24**, C07B 57/00

(22) Date of filing: **21.11.91**

(30) Priority: **29.11.90 JP 325864/90**

(43) Date of publication of application:
**03.06.92 Bulletin 92/23**

(84) Designated Contracting States:
**AT BE CH DE DK ES FR GB GR IT LI LU NL SE**

(71) Applicant: **SANWA KAGAKU KENKYUSHO CO., LTD.**
**No. 35, Higashi-sotobori-cho, Higashi-ku**
**Nagoya, Aichi-ken(JP)**

(72) Inventor: **Kurono, Masayasu, Sanwa Kagaku Kenkyusho Co. Ltd.**
**35, Higashi-sotobori-cho, Higashi-ku**
**Nagoya, Aichi-ken(JP)**
Inventor: **Kondo, Yasuaki, Sanwa Kagaku Kenkyusho Co. Ltd.**
**35, Higashi-sotobori-cho, Higashi-ku**
**Nagoya, Aichi-ken(JP)**
Inventor: **Unno, Ryoichi, Sanwa Kagaku Kenkyusho Co. Ltd.**
**35, Higashi-sotobori-cho, Higashi-ku**
**Nagoya, Aichi-ken(JP)**
Inventor: **Kakigami, Takuji, Sanwa Kagaku Kenkyusho Co. Ltd.**
**35, Higashi-sotobori-cho, Higashi-ku**
**Nagoya, Aichi-ken(JP)**
Inventor: **Sawai, Kiichi, Sanwa Kagaku Kenkyusho Co. Ltd.**
**35, Higashi-sotobori-cho, Higashi-ku**
**Nagoya, Aichi-ken(JP)**

(74) Representative: **Wächtershäuser, Günter, Dr.**
**Tal 29**
**W-8000 München 2(DE)**

(54) **Process for the preparation of optically active 4-oxochroman-2-carboxylic acid derivatives.**

(57) There is described a process for the preparation of optically active 4-oxochroman-2-carboxylic acid derivatives as intermediates for synthesizing hydantoin compounds which shows strong inhibition to aldose reductases to cure incurable complications of diabetes. According to the process, the derivatives are prepared by fractionally recrystallizing a salt of racemic 4-oxochroman-2-carboxylic acid derivative with an optically active secondary amine or diamine.



Rank Xerox (UK) Business Services

The present invention relates to a process for the preparation of optically active 4-oxochroman-2-carboxylic acid derivatives.

The derivatives are shown by the general formula of

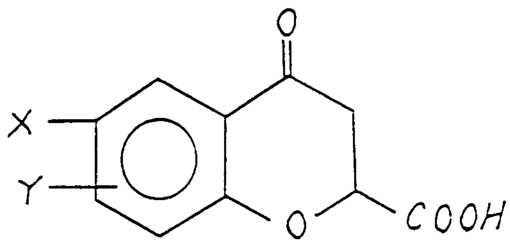

(I)

wherein X and Y are same or different and each represents hydrogen atom, halogen atom or alkyl group.

The derivatives per se have been known and are useful as an intermediate for synthesizing optically active 6-fluoro-2',5'-dioxospiro[chroman-4,4'-imidazolidine]-2-carboxamide which shows strong inhibition to enzyme of aldose reductase is shown by following the formula (V) [see Jap. Pat. No. Hei 1 - 93588(A)]. The compound (V) is great promising as one of effective ingredients for curing complications of diabetes, which are incurable diseases.

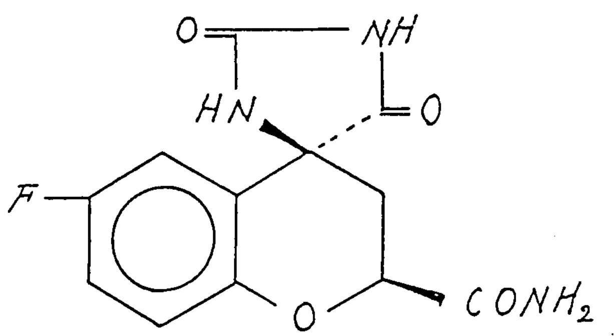

(V)

The compound (V) can be prepared by starting from the 4-oxochroman-2-carboxylicacid derivative (I) as the final product of the process according to the invention, thermally treating the compound (I) [wherein X is fluorine and Y is hydrogen atom] in the presence of sodium cyanide, namely by utilizing a so-called "Bucherer's hydantoin synthesis" to form a hydantoin ring thereto, and then converting the carboxyl radical at 2-position into amid0 radical [see said Jap. Pat. No. Hei 1 - 93588(A)].

The inventors has disclosed one of processes for preparing the compounds (I) in Jap. Pat. No. Sho 63 - 250373(A). According to the process disclosed in the patent literature, the compound has been prepared through following reaction steps.

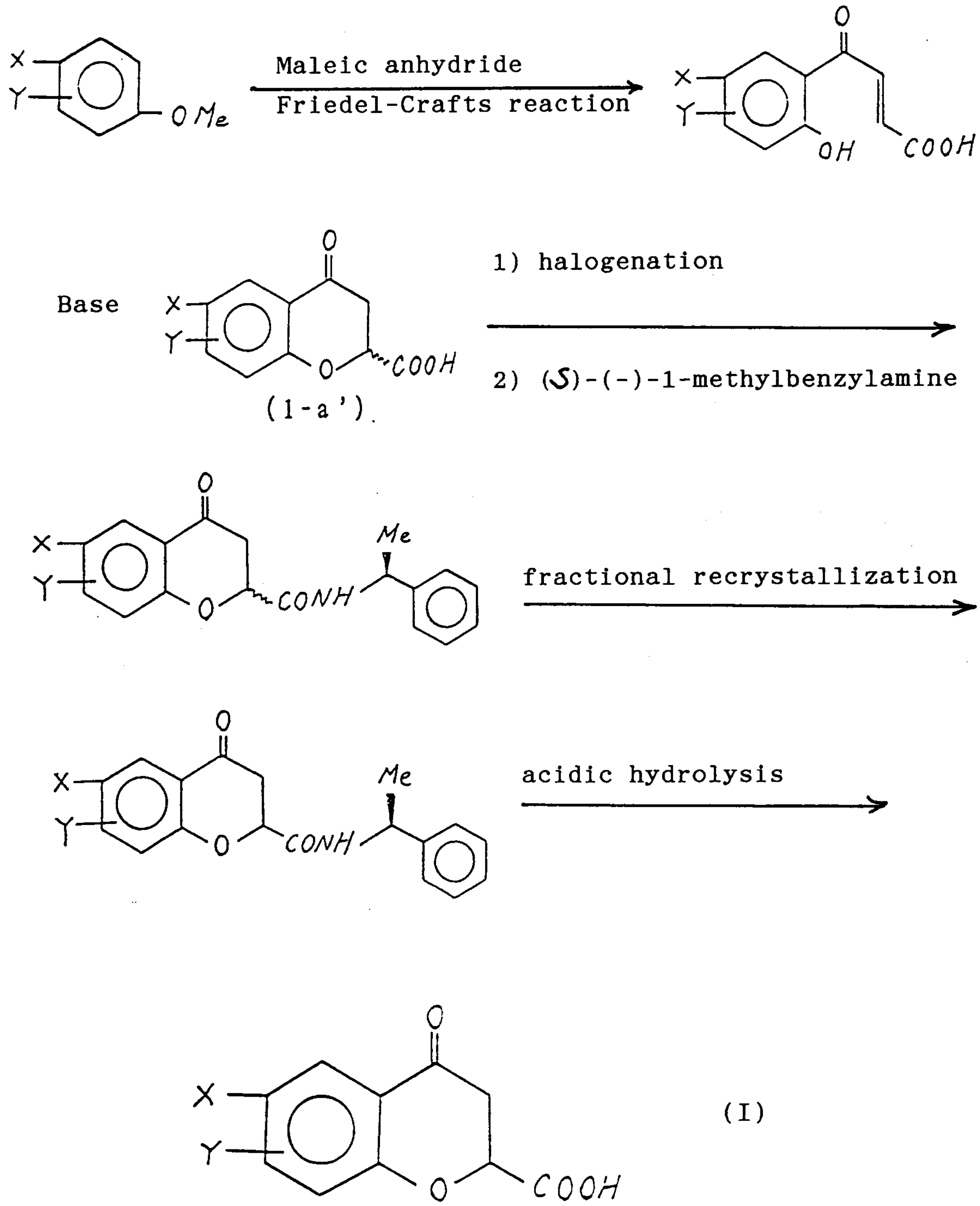


In the above formulae, X and Y have the meanings as referred to, and Me is methyl radical.

For obtaining an optically active compound (I) in accordance with the process disclosed in said Jap. Pat. No. Sho 63 - 250373(A), following steps are required, namely steps of activating racemic 4-oxochroman-2-carboxylic acid derivative (I-a'), reacting the same with ( )-(-)-1-methylbenzylamine to prepare a diastereomer mixture of ( )-(-)-1-methylbenzylamide, fractionally recrystallizing the mixture to obtain (+)-amide derivative, and then acidic hydrolyzing the amide to obtain the desired (+)-carboxylic acid derivative. In other words, this known process consists of many steps comprising the activation of racemic carboxylic acid, amidation, fractional recrystallization and acidic hydrolysis, and thus it can not be always said as the process convenient for carrying out the same in a large scale industrial production.

In case of preparing (+)-4-oxochroman-2-carboxylic acid derivative through a fractional recrystallization of a salt of the racemic 4-oxochroman-2-carboxylic acid derivative (I-a') and an optically active amine, there

are optically active 1-methylbenzylamine, dehydroabiethylamine and the like as resolving reagents which have widely been employed in industrial fields and available with reasonable cost, but those are primary amines and thus each of those reacts with carbonyl radical at 4-position of the 4-oxochroman-2-carboxylic acid derivative to form a Shiff base. Therefore, such optically active primary amines can not be employed as the resolution reagent.

While, quinine, cinchonine and the like natural optically active amines are expensive and thus have the disadvantage from the industrial view point.

Therefore, an object of the invention is to provide a process for the preparation of optically active 4-oxochroman-2-carboxylic acid derivatives (I) with shorter reaction steps and without use any expensive raw material and reagent to allow a large scale industrial production of the derivatives.

The inventors have energetically studied and investigated from economical view points on various conditions inclusive of cost of raw materials, cost of reagents and yield of products and have finally developed a route for synthesizing optically active 4-oxochroman-2-carboxylic acid derivatives, which uses an optically active secondary amine or optically active diamine, as the resolution reagent forming no Shiff base, to establish the invention.

Namely, optically active compounds (I) can be obtained by synthesizing following salt (II) of a racemic 4-oxochroman-2-carboxylic acid derivative and an optically active secondary amine, or a following salt (III) or (IV) of the racemic 4-oxochroman-2-carboxylic acid derivative and an optically active diamine, carrying out a fractionally recrystallization with use of a solvent such as ethanol, isopropanol, diethyl ether, toluene, ethyl acetate or the like, isolating the desired compound (I) as crystals, in a diluted hydrochloric acid solution. The optical purity of product reaches more than 99% e. e.

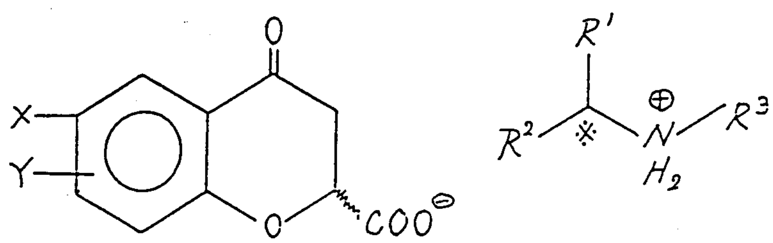

(II)

wherein X and Y is same or different and each represents hydrogen atom, halogen atom or alkyl group, $R^1$ is phenyl radical, substituted phenyl group or naphthyl radical, $R^2$ is hydrogen atom, alkyl group or phenyl radical, $R^3$ is alkyl group, phenyl radical or aralkyl group, but both of $R^1$ and $R^2$ do not concurrently mean the same radical.

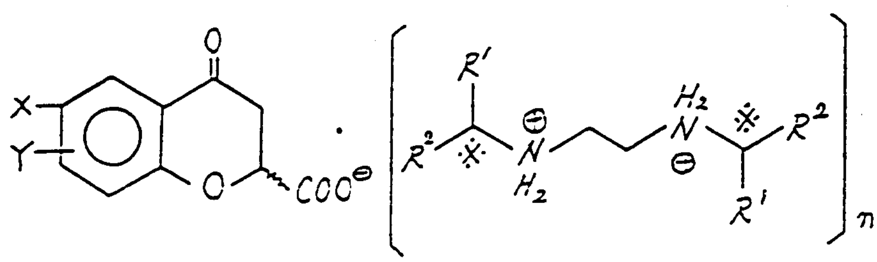

(III)

wherein X, Y, $R^1$ and $R^2$ have the meanings as referred to, and n is 1/2 or 1.

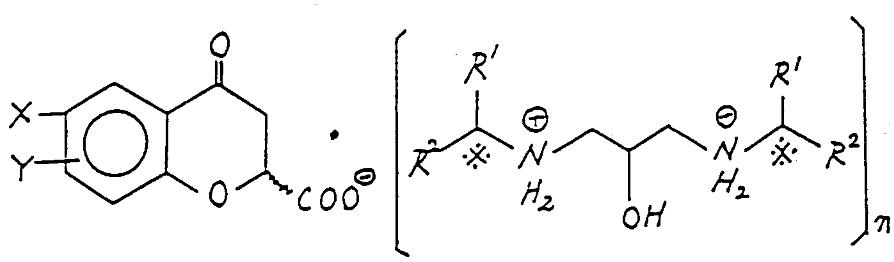

(IV)

wherein X, Y, $R^1$, $R^2$ and n have the meanings as referred to.

The optically active secondary amines to be used as the resolution reagent can be synthesized by a method known per se ("Journal fur Praktische Chemie", [2], 86, 284) or another method similar thereto. Exemplar routes for preparing the secondary amines shall be shown below.

Route A

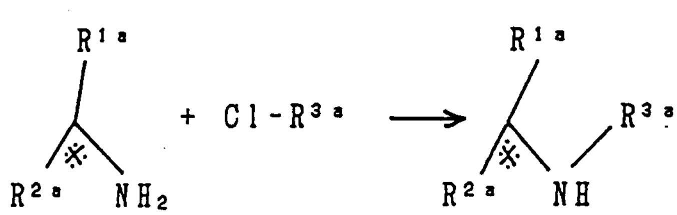

wherein $R^{1a}$ is methyl radical, $R^{2a}$ is phenyl radical and $R^{3a}$ is benzyl radical.

According to this route, the optically active 1-methylbenzylamine is reacted with benzyl chloride to afford the desired amine (see said "Journal fur Praktische Chemie", [2], 86, 284).

Route B

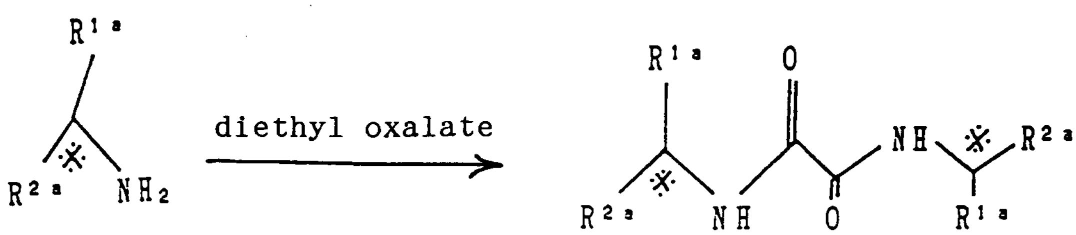


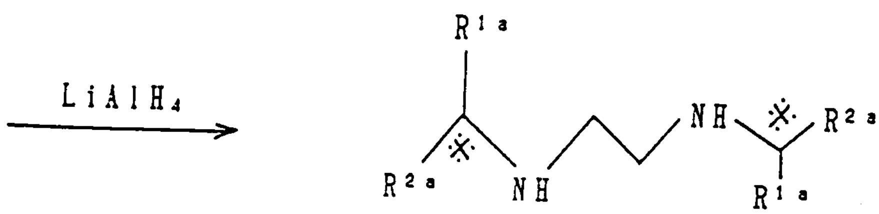


(VI)

wherein $R^{1a}$ and $R^{2a}$ have the meanings as referred to.

According to this route, the optically active 1-methylbenzylamine is reacted with diethyl oxalate and then reduced to afford the desired diamine [see Jap. Pat. No. Sho 52 - 151127(A)].

Route C

Optically active diamines can also be synthesized in accordance with the following reaction.

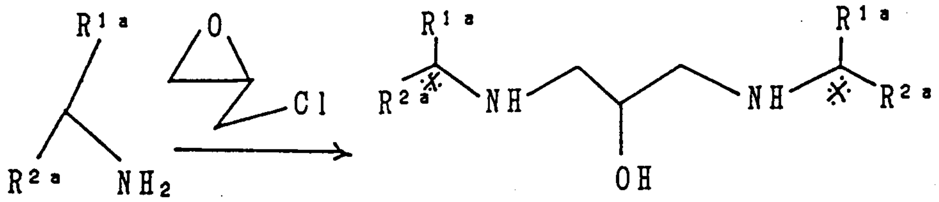

(VII)

wherein $R^{1a}$ and $R^{2a}$ have the meanings as referred to.

The invention will now be further explained in more detail with reference to Reference Examples and Examples.

Reference Example 1

(R)-(+)-N-Benzyl-1-phenylethylammonium hydrochloride

To (R)-(+)-1-methylbenzylamine 38.8g (0.316mol) in toluene 400ml, added benzyl chloride 20.0g (0.158mol) to reflux overnight on an oil bath. After cooled, formed crystals were removed by filtration, and the filtrate was concentrated in vacuo to isolate the hydrogen chloride salt as residue. The residue was recrystallized from ethanol-diethyl ether to afford 24.3g of the desired salt, as colorless crystals (Yield : 62.1%).

Melting point : 178°C (dec.).

Mass spectrum : CI/DI (i-Bu) m/z ;

212 $[(M+1)^+$, base peak].

| $^1$H-NMR spectrum : $CDCl_3$ (ppm) ; | |
|---|---|
| 1.79 | (3H, d, J = 6.83Hz, Me), |
| 3.60 | (1H, d, J = 13.7Hz, N-CH-Ph), |
| 3.87 | (1H, d, J = 13.7Hz, N-CH-Ph), |
| 4.04 | [1H, q, J = 6.83Hz, -CH(Me)-], |
| 7.26 - 7.59 | (10H, m, Ar-H), |
| 10.31 | (2H, br.s, $N^+H_2$). |

$[\alpha]_D^{25}$ : +9.8° (c = 1.00, MeOH).

Reference Example 2

N,N'-Bis[(R)-1-phenylethyl]ethylenediammmonium dihydrochloride

This salt was synthesized in accordance with the method disclosed in Jap. Pat. No. Sho 52 - 151127-(A). Namely, N,N'-bis[(R)-1-phenylethyl]oxalic amide was synthesized with use of (R)-(+)-1-methylbenzylamine 55.0g (0.454mol) and diethyl oxalate 33.1g (0.227mol), and then reduced with lithium aluminum hydride in tetrahydrofuran. The desired salt (41.9g) was obtained as colorless crystals by isolating as hydrogen chloride salt and recrystallizing from ethanol-diethyl ether (Yield : 54.1%).

Melting point : 253°C (dec.).

Mass spectrum : CI/DI (i-Bu) m/z ;

269 $[(M+1)^+$, base peak].

| $^1$H-NMR spectrum : $CDCl_3$ (ppm) ; | |
|---|---|
| 1.80 | (3H x 2, d, J = 6.81Hz, Me), |
| 3.03 - 3.35 | (2H x 2, m, $-CH_2-$), |
| 4.26 | [1H x 2, q, J = 6.81Hz, -CH(Me)-], |
| 7.34 - 7.61 | (14H, m, Ar-H, $N^+H_2$). |

$[\alpha]_D^{27}$ : -26.5° (c = 1.00, MeOH).

Reference Example 3

Bis[(S)-1-phenylethylamino]-2-propanol dihydrochloride

To a solution of (S)-(-)-1-methylbenzylamine 10.0g (82.5mmol) and chloromethyloxirane 3.82g (41.3mmol) in sec-butanol 100ml, added potassium carbonate 5.70g (41.3mmol) to reflux the resulting mixture for 16 hours. The reaction mixture was filtered to remove formed precipitate and the filtrate was concentrated in vacuo. To the residue, 20% hydrogen chloride-diethyl ether solution was added to convert into hydrogen chloride salt and then recrystallized from ethanol to afford 10.2g of the desired salt, as colorless crystals (Yield : 66.7%).
Melting point : 266 ° C (dec.).
Mass spectrum : CI/DI (i-Bu) m/z ;
299 $[(M+1)^+$, base peak].

| $^1$H-NMR spectrum : $CDCl_3$ (ppm) ; | |
|---|---|
| 1.91 | (3H, d, J = 6.83Hz, Me), |
| 1.92 | (3H, d, J = 6.83Hz, Me), |
| 2.47 - 2.90 | (2H x 2, m, $-CH_2-$), |
| 4.23 | [1H, q, J = 6.83Hz, -CH(Me)-], |
| 4.35 | [1H, q, J = 6.83Hz, -CH(Me)-], |
| 4.77 | [1H, t, J = 9.76Hz, -CH(OH)-], |
| 7.33 - 7.61 | (12H, m, Ar-H, $N^+H_2$), |

$[\alpha]_D^{27}$ : -36.9° (c = 1.00, MeOH).

Example 1

(R)-N-Benzyl-1-phenylethyl ammonium (S)-6-fluoro-4-oxochroman-2-carboxylate

(R)-(+)-N-Benzyl-1-phenylethylammonium hydrochloride was reacted with 10% sodium hydroxide to remove hydrochloride from the former. The resulting amine compound 5.03g (23.8mmol) was mixed with racemic 6-fluoro-4-oxochroman-2-carboxylic acid 5.0g (23.8mmol) and fractionally recrystallized from isopropanol to afford 4.40g of the desired compound (Yield : 44.0%).
Melting point : 141 ° C (dec.).
Mass spectrum : CI/DI (i-Bu) m/z ;
212 $[(M+1)^+$, base peak], 210.

IR spectrum : $\nu_{max}^{KBr}$ $(cm^{-1})$ ;

3445 $(NH^+)$, 1697 (C=O), 1628 $(COO^-)$.

| $^1$H-NMR spectrum : $CDCl_3$ (ppm) ; | |
|---|---|
| 1.37 | (3H, d, J = 6.83Hz, Me), |
| 2.94 | (1H x 2, dd, J = 8.28Hz, 17.1Hz, CO-CH-), |
| 3.05 | (1H x 2, dd, J = 4.89Hz, 17.1Hz, CO-CH-), |
| 3.61 | (1H, d, J = 13.2Hz, N-CH-Ph), |
| 3.68 | (1H, d, J = 13.2Hz, N-CH-Ph), |
| 3.97 | [1H x 2, q, J = 6.83Hz, -CH(Me)-], |
| 4.85 | (1H, dd, J = 4.89Hz, 6.83Hz, -O-CH-), |
| 6.91 - 7.55 | (13H, m, Ar-H). |

| Elementary analysis ($C_{25}H_{25}FNO_4$) : | | | |
|---|---|---|---|
| Cal. | C = 71.25, | H = 5.74, | N = 3.32. |
| Found | C = 71.42, | H = 5.69, | N = 3.30. |

$[\alpha]_D^{26}$ : +33.6° (c = 1.00, MeOH).

Example 2

N,N'-Bis-[( )-1-phenylethyl]ethylenediammonium bis( )-6-fluoro-4-oxochroman-2-carboxylate

N,N'-Bis[( )-1-phenylethyl]ethylenediammonium dihydrochloride was reacted with 10% sodium hydroxide to remove hydrochloride from the former. The resulting diamine compound 3.16g (11.9mmol) was mixed with racemic 6-fluoro-4-oxochroman-2-carboxylic acid 5.0g (23.8mmol) and fractionally recrystallized from isopropanol to afford 2.87g of the desired compound (Yield : 35.0%).
Melting point : 152° C (dec.).
Mass spectrum : CI/DI (i-Bu) m/z ;
269[$(M+1)^+$, base peak], 210.

IR spectrum : $\nu_{max}^{KBr}$ ($cm^{-1}$) ;

3445 ($NH^+$), 1697 (C=O), 1628 ($COO^-$).

| $^1$H-NMR spectrum : $CDCl_3$ (ppm) ; | |
|---|---|
| 1.63 | (3H x 2, d, J = 6.83Hz, Me), |
| 2.55 - 2.63 | (1H x 2, m, -CH-N), |
| 2.90 - 2.98 | (1H x 2, m, -CH-N), |
| 3.07 | (1H x 2, dd, J = 7.81Hz, 17.1Hz, CO-CH-), |
| 3.15 | (1H x 2, dd, J = 5.37Hz, 17.1Hz, CO-CH-), |
| 3.89 | [1H x 2, q, J = 6.83Hz, -CH(Me)-], |
| 4.99 | (1H, dd, J = 5.37Hz, 7.81Hz, -O-CH-), |
| 7.01 - 7.55 | (16H, m, Ar-H). |

| Elementary analysis ($C_{38}H_{38}F_2N_2O_8$) : | | | |
|---|---|---|---|
| Cal. | C = 66.27, | H = 5.56, | N = 4.07. |
| Found | C = 66.01, | H = 5.73, | N = 4.17. |

$[\alpha]_D^{26}$ : +65.5° (c = 1.01, MeOH).

Example 3

2-Hydroxy-N,N'-bis-[(S)-1-phenylethyl]propylenediammonium bis(S)-6-fluoro-4-oxochroman-2-carboxylate

Procedures similar to Example 2 were carried out, excepting that such an amine compound 3.55g (11.9mmol) was employed, as obtained by treating bis[(S)-1-phenylethylamino]-2-propanol dihydrochloride instead of N,N'-bis[(R)-1-phenylethyl]ethylenediammonium dihydrochloride with 10% sodium hydroxide to remove hydrochloride from the former, to afford 2.73g of the desired compound (Yield : 31.9%).
Melting point : 190° C (dec.).
Mass spectrum : CI/DI (i-Bu) m/z ;
299$(M+1)^+$, base peak], 210.

IR spectrum : $\nu_{max}^{KBr}$ ($cm^{-1}$) ;

3445 ($NH^+$), 1695 (C=O), 1628 ($COO^-$).

| $^1$H-NMR spectrum : DMSO-$d_6$ (ppm) ; | |
|---|---|
| 1.33 | (3H, d, J = 6.84Hz, Me), |
| 1.36 | (3H, d, J = 6.84Hz, Me), |
| 2.24 - 2.72 | (2H x 2, m, -$CH_2$), |
| 2.94 | (1H x 2, dd, J = 6.35Hz, 17.1Hz, -CH-N), |
| 3.06 | (1H x 2, dd, J = 5.37Hz, 17.1Hz, -CH-N), |
| 3.99 | [1H x 2, q, J = 6.84Hz, -CH(Me)-], |
| 3.87 - 4.06 | [1H, m, -CH(OH)-], |
| 4.98 | (1H x 2, dd, J = 5.37Hz, 6.35Hz, -O-CH-), |
| 7.09 - 7.46 | (16H, m, Ar-H). |

| Elementary analysis ($C_{39}H_{40}F_2N_2O_9$) : | | | |
|---|---|---|---|
| Cal. | C = 65.17, | H = 5.61, | N = 3.90. |
| Found | C = 65.33, | H = 5.73, | N = 3.88. |

$[\alpha]_D^{26}$ : +26.8° (c = 0.50, MeOH).

Example 4

(S)-6-Fluoro-4-oxochroman-2-carboxylic acid

To (R)-N-benzyl-1-phenylethylammonium (S)-6-fluoro-4-oxochroman-2-carboxylate 2.00g (4.75mmol) in hot water 20ml, added concentrated hydrochloric acid 1.0ml, and then cooled to 5 °C. The reaction mixture was filtered to obtain crystals which were washed with chilled water, dried in vacuo to afford 0.829g of the desired optically active carboxylic acid (Yield : 83.1%).
Melting point : 176°C (dec.).
Mass spectrum : EI/DI (i-Bu) m/z ;
210($M^+$), 165 (base peak).

IR spectrum : $\nu_{max}^{KBr}$ ($cm^{-1}$) ;

2200 - 3400 (OH), 1738 (COOH), 1666(C=O), 1627, 1617, 1489, 1444 (C=C, Aromatic ring).

| $^1$H-NMR spectrum : DMSO-$d_6$ (ppm) ; | |
|---|---|
| 2.98 | (1H, dd, J = 17.1Hz, 7.8Hz, $C_3$-H), |
| 3.12 | (1H, dd, J = 17.1Hz, 5.4Hz, $C_3$-H), |
| 5.32 | (1H, dd, J = 7.3Hz, 5.4Hz, $C_2$-H), |
| 7.17 | (1H, dd, J = 8.8Hz, 4.4Hz, $C_8$-H), |
| 7.42 | (1H, dd, J = 8.8Hz, 3.4Hz, $C_5$-H), |
| 7.47 | (1H, dt, J = 8.8Hz, 3.4Hz, $C_7$-H), |
| 13.34 | (1H, br.s, OH). |

| Elementary analysis ($C_{18}H_7FO_4$) : | | |
|---|---|---|
| Cal. | C = 57.15, | H = 3.36. |
| Found | C = 57.35, | H = 3.34. |

$[\alpha]_D^{26}$ +61.4° (c = 1.00, MeOH).

Example 5

(S)-6-Fluoro-4-oxochroman-2-carboxylic acid

Procedures described in Example 4 were carried out, excepting that 2.00g (5.81 mmol) of N,N'-bis[(R)-1-phenylethyl]ethylenediammonium bis( )-6-fluoro-4-oxochroman-2-carboxylate was employed instead of (R)-N-benzyl-1-phenylethylammonium (S)-6-fluoro-4-oxochroman-2-carboxylate , to afford 1.04g of the desired compound (Yield : 85.2%).
Melting point : 176° C (dec.).

Example 6

(S)-6-Fluoro-4-oxochroman-2-carboxylic acid

Procedures described in Example 4 were carried out, excepting that 2.00g (5.57 mmol) of 2-hydroxy-N,N'-bis[(S)-1-phenylethyl]propylene diammonium bis (S)-6-fluoro-4-oxochroman-2-carboxylate was employed instead of (R)-N-benzyl-1-phenylethylammonium (S)-6-fluoro-4-oxochroman-2-carboxylate, to afford 0.99g of the desired compound (Yield : 84.6%).
Melting point : 175 - 176° C (dec.).

**Claims**

**1.** A process for the preparation of optically active 4-oxochroman-2-carboxylic acid derivatives of the formula

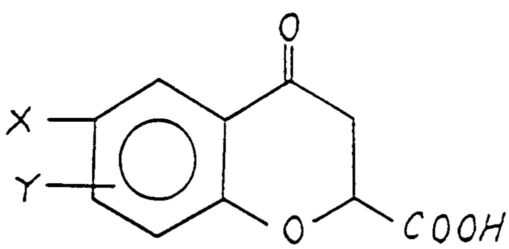

(I)

wherein X and Y are same or different and each represents hydrogen atom, halogen atom or alkyl group,
which comprises a step of fractionally recrystallizing an optically active compound of the formula

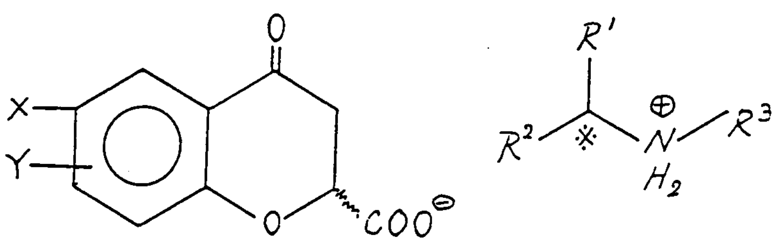

(II)

wherein X and Y have the meanings as referred to, $R^1$ is phenyl radical, substituted phenyl group or naphthyl radical, $R^2$ is hydrogen atom, alkyl group or phenyl radical, $R^3$ is alkyl group, phenyl radical or aralkyl group, but both of $R^1$ and $R^2$ do not concurrently mean same radical,
to isolate the carboxylic acid derivative.

**2.** A process for the preparation of optically active 4-oxochroman-2-carboxylic acid derivatives of the formula

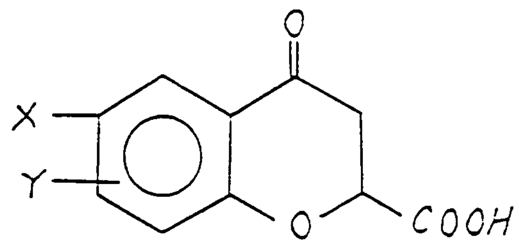

(I)

wherein X and Y are same or different and each represents hydrogen atom, halogen atom or alkyl group,
which comprises a step of fractionally recrystallizing an optically active compound of the formula

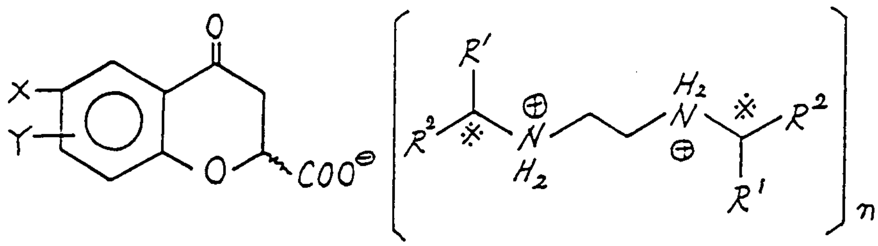

(III)

wherein X and Y have the meanings as referred to, $R^1$ is phenyl radical, substituted phenyl group or naphthyl radical, $R^2$ is hydrogen atom, alkyl group or phenyl radical, but both of $R^1$ and $R^2$ do not concurrently mean same radical, and n is 1/2 or 1,
to isolate the carboxylic acid derivative.

3. A process for the preparation of optically active 4-oxochroman-2-carboxylic acid derivatives of the formula

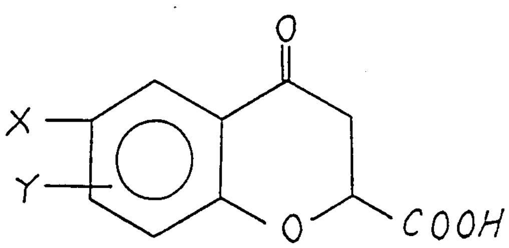

(I)

wherein X and Y are same or different and each represents hydrogen atom, halogen atom or alkyl group,
which comprises a step of fractionally recrystallizing an optically active compound of the formula

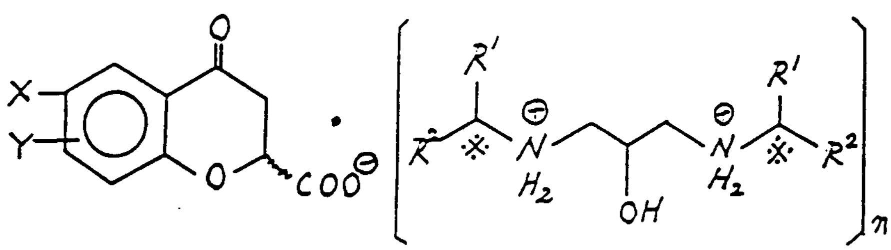

(IV)

wherein X and Y have the meanings as referred to, $R^1$ is phenyl radical, substituted phenyl group or naphthyl radical, $R^2$ is hydrogen atom, alkyl group or phenyl radical, but both of $R^1$ and $R^2$ do not

concurrently mean same radical, and n is 1/2 or 1, to isolate the carboxylic acid derivative.

European Patent Office

# EUROPEAN SEARCH REPORT

Application Number

EP 91 11 9844

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A<br><br>D | EP-A-0 264 586 (SANWA)<br>* page 13 - page 14; examples 3,6,24 *<br>& JP-A-63 250 373 (SANWA)<br>--- | 1-3 | C07D311/24<br>C07B57/00 |
| A | EP-A-0 035 811 (OCE-ANDENO)<br>* page 1 - page 2 *<br>--- | 1 | |
| A | LIEBIGS ANNALEN DER CHEMIE,<br>no. 6, 12 June 1984, Weinheim, DE,<br>pages 1240 - 1257;<br>L. HORNER ET AL.: 'Die Reduktion prochiraler Alkyl(aryl)ketone zu carbinolen und pinakolen mit Alkaliamalgamen'<br>* compound 8 *<br>----- | 2 | |

TECHNICAL FIELDS SEARCHED (Int. Cl.5)

C07D
C07B
C07C

The present search report has been drawn up for all claims

| Place of search | Date of completion of the search | Examiner |
|---|---|---|
| THE HAGUE | 11 FEBRUARY 1992 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)